# EUROPEAN PATENT APPLICATION

(11) **EP 1 182 190 A1**
(43) Date of publication of application: **27.02.2002**
(21) Application number: 00810759.1
(22) Date of filing: 25.08.2000
(51) Int. Cl.: C07C 69/608, C07C 69/753, C11B 9/00, A61K 7/46

(54) **Unsaturated esters**

(71) Applicant: Givaudan SA, 1214 Vernier-Genève (CH)
(72) Inventor: Goeke, Andreas, 8600 Dübendorf (CH); Berg-Schulz, Katja, 4303 Kaiseraugst (CH); Bajgrowicz, Jerzy, 8053 Zürich (CH)
(74) Representative: Patentanwälte Schaad, Balass, Menzl & Partner AG

(57) **Abstract**

The present invention relates to new α,β-unsaturated esters of formula I. The new compounds exhibit an intense, very long lasting fruity-pineapple odor with green galbanum-type undertones.

## Description

The present invention relates to new α,β-unsaturated esters and to their use.

Inexpensive floral fruity and green fragrances with intense pineapple and galbanum undertones are highly desirable in the art of perfumery. Many of the natural and commercially available compounds are expensive and show lack of stability. Moderately weak galbanum type compounds are available including allyl amyl glycolate (International Flavors and Fragrances), Cyclogalbanate® (Dragoco)and allyl cyclopentyl glycolate, the latter is described in the patent specification US 4,735,932.

1- (5,5-dimethyl-1-cyclohexen-1-yl)-4-pentene-1-one has been described by Morris, A.F. et al. in Perfumer & Flavorist **1991,** (16), 33, as an important compound for the perfumery, which exhibits a powerful metallic odor reminiscent of galbanum with pineapple and hyacinth character. Said compound adds fresh, green, floral and fruity aspects to perfumes and perfumed products.

In the yet unpublished European patent application with the application number EP 105 851 α,β-unsaturated ketones are described.

It is an object of the present invention to provide new compounds which have a precious and long lasting fruity, mainly pineapple type of odor accompanied by green and intense galbanum undertones.

Surprisingly it has been found that an intense, very long lasting fruity-pineapple odor with galbanum type undertones is exhibited by compounds of the formula I 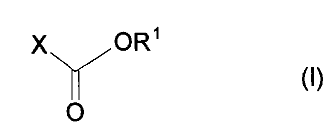 wherein R¹ is a straight or branched C₃-C₄ alkenyl residue having the double bond in position 2 or a C₄ alkylcycloalkyl residue , i.e. allyl, methallyl, crotyl or methylcyclopropyl residue, and
X is either a residue of formula A 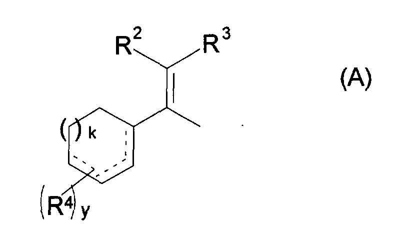
wherein
R², R³ are independently hydrogen, methyl or ethyl,
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k is 0 or an integer of 1,2 or 3,
and the dotted lines in formula A stand for one optional double bond,
or X is a residue of formula B 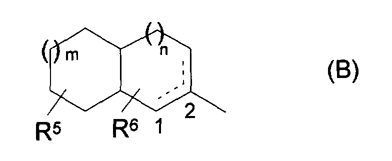 wherein
R⁵ or R⁶ are independently hydrogen or a straight or branched C₁-C₃ hydrocarbon residue being at any position of the ring system B, and the sum of the carbon atoms of R⁵ and R⁶ is 3 or less,
n is 0 or an integer of 1 or 2,
m is 0 or an integer of 1 or 2,
and the dotted lines in formula B stand for an optional double bond either in position 1 or 2.

The above formulas include all different possible stereo-and double-bond isomers.

### New compounds of formula Ia are preferred

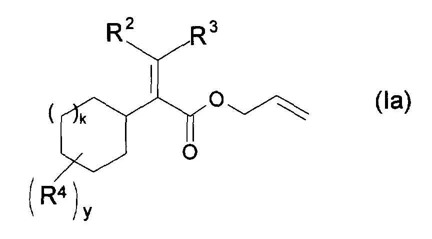

wherein
R², R³ are independently hydrogen or methyl
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k stands for 0 or 1.

Further preferred compounds of formula (I) are such
wherein R¹ is C₄ alkylcycloalkyl (such as methylcyclopropyl) or a C₄ alkenyl residue (such as crotyl) having the double bond in position 2,
and
X is a residue of formula (A) 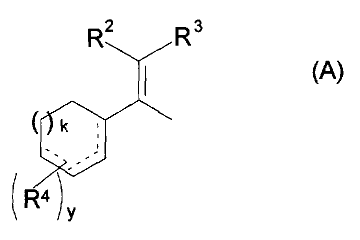
wherein
R² and R³ are independently hydrogen or methyl,
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k is 0 or 1.

The new α,β-unsaturated esters of formula I, wherein X is a residue of formula A, may be prepared according to procedures known in the art as shown in scheme 1.

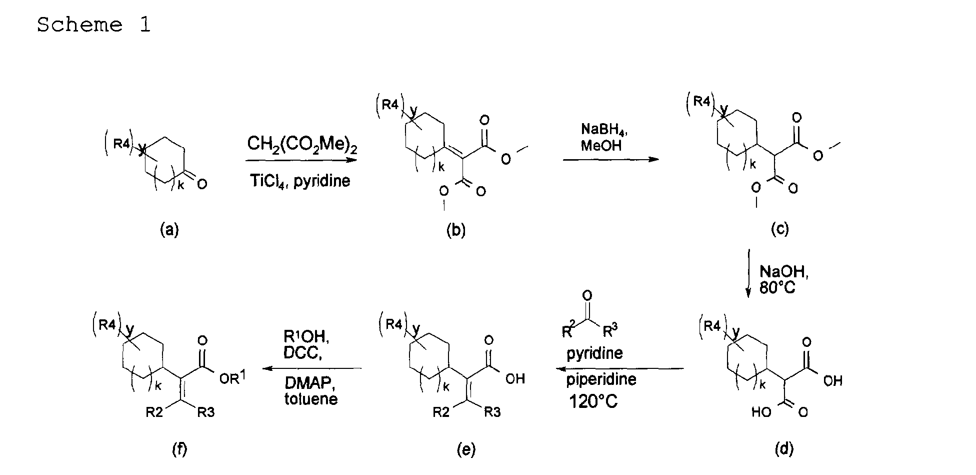

The starting ketones (a) may be converted by a Knoevenagel reaction well known in the art to compounds (b) . The α,β-unsaturated diesters may be reduced by metal hydrides or hydrogen to (c), and then saponified to (d) . Diacids of type (d) may then be decarboxylated and in situ methylenated to α,β-unsaturated mono acids of structure (e) (R², R³ = H) which can be esterified under a variety of conditions known to the chemist.

Alternatively, alkylidene units may be introduced as depicted in scheme 2: Condensation of an allylester of structure (g) with an aldehyde or ketone in the presence of LDA followed by dehydration of the intermediate alcohols (h) (R², R³ = H, alkyl) to the desired esters (f) .

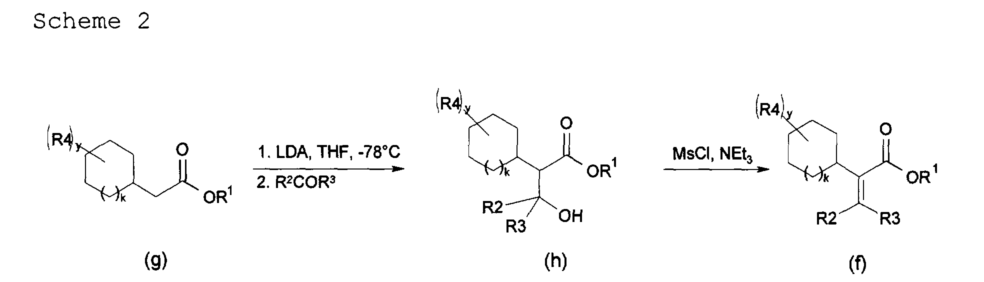

The new **α**,β-unsaturated esters of formula I wherein X is a residue of formula B can be prepared from the corresponding bicyclic ketones according to the process illustrated in scheme 3.

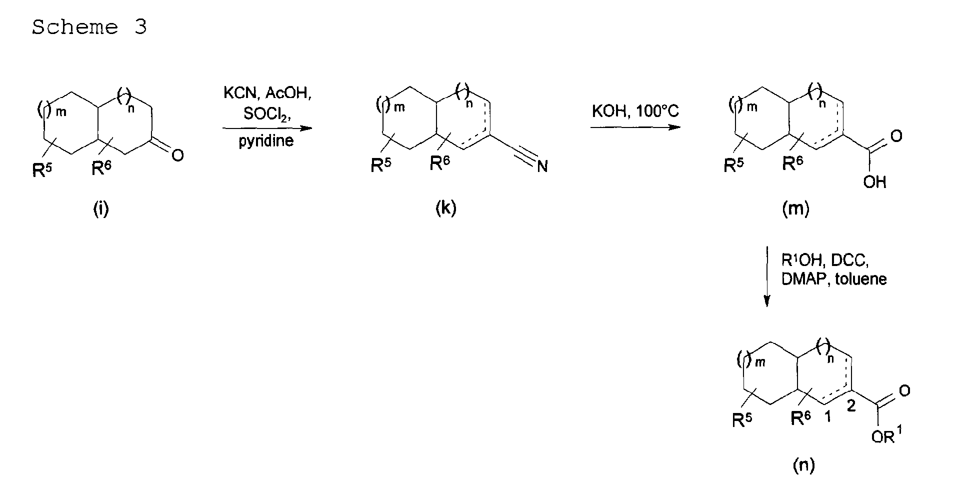

The α,β-unsaturated esters wherein X is a residue of formula B, are generally obtained as mixtures of cis/trans-isomers. The double bond is either in position 1 or 2.

Compositions comprising a compound according to the present invention possess fruity organolepic characteristics. The above mentioned organoleptic characteristics make the new compounds well suited for different functional perfumery applications, as well as for imparting unique green galbanum and fruity notes to fine perfumery products. In addition to said excellent characteristics, the compounds of the invention show outstanding diffusion and/or high substantivity, the latter meaning persistence of odor. The high diffusion and substantivity is well perceived on fabrics washed with a detergent or treated with a softener comprising one or more of the new α,β-unsaturated esters. The typical fresh green odor is already perceived very strongly on the wet fabric and later also on the dry material.

Due to the excellent odor and application qualities, the new compounds are excellent fragrances for use in any field of fine and functional perfumery, such as perfumes, household products, laundry products, body care products and cosmetics.

Fragrance or flavor compositions comprising a compound according to the present invention may optionally be combined with numerous organoleptic ingredients of natural and/or synthetic origin. The range of the natural fragrances and flavors includes in addition to readily volatile, also moderately and only slightly volatile components. The synthetic organoleptic ingredients embrace representatives from practically all classes of organoleptic substances. The following list comprises examples of known organoleptic ingredients which may be combined with the compounds of the invention:
natural products: tree moss absolute, basil oil, tropical fruit oils (such as bergamot oil, mandarin oil, etc.), mastix absolute, myrtle oil, palmarosa oil, patchouli oil, petitgrain oil, wormwood oil, lavender oil, rose oil, jasmin oil, ylang-ylang oil, etc.;
alcohols: farnesol, geraniol, linalool, nerol, phenylethyl alcohol, rhodinol, cinnamic alcohol, (Z)-hex-3-en-1-ol, menthol, α-terpineol, etc.;
aldehydes: citral, α-hexyl cinnamaldehyde, Lilial, methylionone, verbenone, nootkatone, geranylacetone, etc.;
esters: allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, methyl dihydrojasmonate, styralyl propionate, vetiveryl acetate, benzyl acetate, geranyl acetate, etc.;
lactones: γ-undecalactone, δ-decalactone, pentadecanolide, 12-oxahexadecanolide, etc.;
acetals: Viridine (phenylacetaldehyde dimethylacetal), etc.;
other components often used in perfumery: indole, p-mentha-8-thiol-3-one, methyleugenol, eugenol, anethol, etc..

The novel compounds of the invention harmonize particularly well with floral notes (lily of the valley, rose, iris, jasmine, ylang-ylang, narcissus notes, etc.) as well as with woody, chypre and animalic notes, tobacco like an patchouli compositions, etc.

The percentage in which the compounds of the invention are used in a composition may vary within wide limits ranging from a few parts per thousand in mass market products (e.g. cleaning compositions, deodorant, etc.) up to a few percents in alcoholic extracts for fine perfumery. In all cases, even in small amounts, the compounds of formula I provide fragrance compositions with green-galbanum and intense fresh green-fruity notes and a remarkable increase of the volume (strength, diffusivity) and of the duration (substantivity) of the odor.

There is no restriction regarding the type of formulations and the destination of the actual finished product: eau de cologne, toilet water, scented water, perfume, body care and cosmetic products such as cream, shampoo, soap, household products such as detergent, household cleaner, fabric softener, etc., come into consideration.

The organoleptic characteristic of compounds according to the present invention make the new compounds well suited for imparting an intense pineapple and galbanum flavor into food and beverage products.

The invention will be further described, by way of illustration, in the following examples.

All compounds were unambiguously identified by their ¹H-NMR- (chemical shifts (δ) are given in ppm downfield from TMS; coupling constants *J* in Hz),IR- and MS-spectra.

For the exact definition of the trivial names mentioned above and in the examples, see Flavor and Fragrance materials 1998, Allured publishing Corporation, Carol Stream, Illinois, U.S.A. or Arctander, Perfume and Flavor Chemicals - 1969, published by the author, Montclair, New Jersey, U.S.A.

### Example 1

### 2-Cyclohexyl-acrylic acid allyl ester

### a) Cyclohexylidene-malonic acid dimethyl ester

Dimethyl malonate (45g, 340 mmol), cyclohexanone (32g, 328 mmol) and pyridine (110 ml, 1.36 mol) were added sequentially to a solution of TiCl₄ (75 ml, 680 mmol) in THF/CH₂Cl₂ (1:3.2) at 0°C. After 0.5h, the reaction was allowed to reach room temperature and was kept stirring over night. Water (420 ml) was added and the mixture was extracted with CH₂Cl₂ (2 x 400 ml). The organic phase was washed with saturated NaHCO₃, water and brine, dried (MgSO₄) and concentrated *in vacuo.* The crude product was distilled (bp 96°C/0.6Torr) to yield 41.7g (60%) of a colorless liquid. ¹H-NMR (200 MHz, CDCl₃) : 3.75 (s, 6H, CO₂CH₃), 2.52-2.47 (m, 4H), 1.75-1.51 (m, 6H) ppm.

### b) Cyclohexyl-malonic acid dimethyl ester

A solution of sodium borohydride (3.78g, 96 mmol) in ethanol (45 ml) was added dropwise to a mixture of cyclohexylidene-malonic acid dimethyl ester in ethanol (80 ml) at 0°C. After 7.5 h an additional amount of hydride (1g) was added. 2 h later, the reaction was quenched by slow addition of 10% HCl (pH ∼1) and the reaction mixture was diluted with CH₂Cl₂ (200 ml). The organic phase was separated and the aqueous phase was extracted with CH₂Cl₂. The combined organic layers were washed with brine, dried (MgSO₄) and concentrated *in vacuo.* The residue was distilled (bp. 84°C, 0.3 Torr) to yield 24.7g (60%) of an colorless oil. ¹H-NMR (200 MHz, CDCl₃): 3.73 (s, 6H, CO₂CH₃), 3.15 (d, 1H, C*H*(CO₂CH₃)₂), 2.17-1.98 (m, 1H) , 1.49-1.01 (m, 10H) ppm.

### c) Cyclohexyl-malonic acid

A suspension of the above diester (19g, 88.8 mmol) in aqueous NaOH (140 ml, 4.4 M) was stirred at 80°C for 6 h. The mixture was cooled to room temperature and acidified with HCl to pH = 1. The solution was concentrated under reduced pressure until a solid precipitated. The solid was filtered off and the filtrate was extracted 3 times with ethyl acetate. The organic phases were combined with the solid and the solution was dried (MgSO₄) and concentrated *in vacuo* to give 14.8 g (90%) of the diacid which was used in the next step without further purification.

### d) 2-Cyclohexyl-acrylic acid methyl ester

Piperidine (2.1ml, 79.6 mmol) and paraformaldehyde (7.7g, 266 mmol) were added to a solution of cyclohexyl-malonic acid (14.8g, 79.6 mmol) in pyridine (85 ml). The mixture was heated at 120°C for 40 min. Pyridine was then evaporated *in vacuo* and the mixture was acidified with conc. HCl. The resulting solution was extracted with diethyl ether (3 X 100 ml) and the organic phase was washed with brine, dried (MgSO₄) and concentrated *in vacuo.* The residue was purified by chromatography on silica (CH₂Cl₂:methanol = 97:3) to afford 10.7g (80%) of a colorless oil. 1H-NMR (200 MHz, CDCl₃): 6.25 (s, 1H, C=C*Ha*Hb), 5.58 (s, 1H, C=CHa*Hb*), 2.41 (m, 1H, 1'-H), 1.84-1.74 (m, 5H), 1.34-1.31 (m, 2H), 1.17-1.10 (m, 3H) ppm.

### e) 2-Cyclohexyl-acrylic acid allyl ester

### Odor: linear, fruity, galbanone, pineapple, green

A solution of 2-cyclohexyl-acrylic acid (2.9g, 19.2 mmol)in toluene (19 ml) was added to a mixture of allyl alcohol (1.7 ml, 23 mmol), dicyclohexyl carbodiimide (DCC, 4.74g, 23 mmol) and 4-dimethyl aminopyridine (DMAP, 2.8g, 23 mmol) in toluene (40 ml). After 12 h the reaction mixture was passed through a plug of silica, was eluated with CH₂Cl₂ and concentrated *in vacuo.* The crude material was purified by chromatography on silica (hexane:CH₂Cl₂ = 70:30) to yield 2.7g (74%) of a colorless oil. ¹H-NMR (400 MHz, CDCl₃): 6.15 (d, *J*_{*3a,3b*} *=* 1Hz, 1H, 3-Ha), 5.94 (ddt, *J* = 17.2, 10.4, 5.6 Hz, 1H, OCH₂C*H*=CH₂), 5.50 (dd, *J*_{*3b,3a*} = 1Hz, *J*_{*3b,1'*} *=* 1.3 Hz, 1H, 3-Hb), 5.34 (ddt, *J* = 17.2 (*trans*), 1.5, 1.5 Hz, 1H, OCH₂CH=C*Ha*Hb), 5.24 (ddt, *J* = 10.4 (*cis*), 1.5, 1.5 Hz, 1H, OCH₂CH=CHa*Hb*), 4.66 (ddd, *J* = 5.6, 1.5, 1.5 Hz, 2H, OCH₂), 2.47 (ttd, *J* = 11.6, 3.3, 1.3 Hz, 1H, 1'-H), 1.85-1.71 (m, 5H), 1.41-1.29 (m, 2H), 1.24-1.06 (m, 3H) ppm. MS(EI): 194 (M⁺, 3), 165 (2), 153 (20), 135 (56), 117 (19), 107 (65), 81 (42), 78 (84), 67 (100). IR (ATR): 2926s, 2853m, 1717vs, 1449m, 1274s, 1231s, 1150vs, 1116s, 984m, 936m cm⁻¹.

### Example 2

### 2-Cyclohexyl-acrylic acid methallyl ester

### Odor: fruity, pineapple, galbanone, green, floral

¹H-NMR (400 MHz, CDCl₃): 6.14 (s, 1H, 3-Ha), 5.48 (s, 1H, 3-Hb), 4.98 (s, 1H, OCH₂C(CH₃)=C*Ha*), 4.92 (s, 1H, OCH₂C(CH₃)=C*Hb*), 4.56 (s, 2H, OCH₂), 2.47-2.40 (m, 1H, 1'-H), 1.83-1.67 (m, 5H) , 1.74 (s, 3H, OCH₂C(*CH*_{*3*})=CH₂), 1.37-1.27 (m, 2H), 1.19-1.04 (m, 3H) ppm. MS(EI): 208 (M⁺, 1), 163 (2), 137 (100), 135 (17), 107 (39), 81 (24), 78 (32), 67 (35).

### Example 3

### 2-Cyclohexyl-acrylic acid crotyl ester

### Odor: galbanone, fruity, green

¹H-NMR (400 MHz, CDCl₃): 6.09 (d, *J* = 0.9 Hz, 1H, 3-Ha), 5.82-5.74 (m, 1H, OCH₂C*H*=CHCH₃), 5.63-5.55 (m, 1H, OCH₂CH=C*H*CH₃), 4.56 (d, *J* = 5.9 Hz, 2H, OCH₂), 2.47-2.39 (m, 1H, 1'-H), 1.81-1.70 (m, 8H), 1.39-1.28 (m, 2H), 1.19-1.05 (m, 3H) ppm. MS(EI): 208 (M⁺, 3), 179 (11), 154 (12), 135 (32), 126 (31), 107 (38), 81 (46), 798 (53), 67 (100).

### Example 4

### 2-Cyclohexyl-acrylic acid methylcyclopropyl ester

### Odor: galbanone, fruity, pineapple, green

¹H-NMR (400 MHz, CDCl₃): 6.11 (s, 1H, 3-Ha), 5.45 (s, 1H, 3-Hb), 3.97 (d, *J* = 7.2 Hz, 2H, OCH₂), 2.46-2.40 (m, 1H, 1'-H), 1.81-1.65 (m, 5H), 1.39-1.27 (m, 2H), 1.21-1.06 (m, 4H), 0.55-0.52 (m, 2H), 0.29-0.26 (m, 2H) ppm. MS(EI): 208 (M⁺, 28), 193 (19), 165 (16), 137 (100), 135 (16), 109 (31), 107 (28), 81 (34), 79 (46), 67 (66).

### Example 5

### 2-Cyclohexyl-but-2-enoic acid allyl ester

### Odor: galbanone, dynascone, fruity, green

¹H-NMR (400 MHz, CDCl₃) : 6.68 (q, *J*_{*3,4*} = 7.1 Hz, 1H, 3-H), 5.97-5.85 (m, 1H, OCH₂C*H*=CH₂), 5.31 (d, *J* = 16.7 Hz, 1H, OCH₂CH=C*Ha*Hb), 5.21 (d, *J* = 10.5 Hz, 1H, OCH₂CH=CHa*Hb*), 4.59 (d, *J* = 5.5 Hz, 2H, OCH₂), 2.54-2.45 (m, 1H, 1'-H), 1.85-1.65 (m, 5H) , 1.79 (d, *J*_{*4,3*} = 7.1 Hz, 3H, 4-H), 1.52-1.50 (m, 2H), 1.31-1.10 (m, 3H) ppm. MS(EI): 208 (M⁺, 9), 167 (50), 149 (75), 121 (41), 107 (19), 93 (62), 81 (100), 79 (86), 67 (91).

### Example 6

### 2-Cyclohexyl-pent-2-enoic acid allyl ester

### Odor: galbanone-like, fruity

¹H-NMR (400 MHz, CDCl₃) : 6.57 (t, *J*_{*3,4*} *=* 7.3 Hz, 1H, 3-H), 5.99-5.88 (m, 1H, OCH₂C*H*=CH₂), 5.32 (d, *J* = 17.0 Hz, 1H, OCH₂CH=C*Ha*Hb), 5.21 (d, *J* = 9.6 Hz, 1H, OCH₂CH=Ha*Hb*), 4.66-4.54 (m, 2H, OCH₂), 2.51-2.44 (m, 1H, 1'-H), 2.19 (dq, *J*_{*4,3*} = 7.3, *J*_{*4,5*} = 7.5 Hz, 2H, 4-H), 1.82.1.60 (m, 5H), 1.51-1.48 (m, 2H), 1.28-1.11 (m, 3H), 1.03 (t, *J*_{*5,4*} = 7.5 Hz, 3H, 5-H) ppm. MS(EI): 222(M⁺, 10), 193 (11), 181 (74), 163 (68), 135 (57), 107 (27), 95 (61), 81 (91), 79 (100), 67 (98).

### Example 7

### 2-(4-Ethyl-cyclohexyl)-acrylic acid allyl ester

### Odor: fruity, green, rosy, galbanone

Two isomers in a ratio of 3/2: ¹H-NMR (400 MHz, CDCl₃): 6.15 (s, 1H, 3-Ha), 6.01-5.91 (m, 1H, OCH₂C*H*=CH₂), 5.53/5.50 (2s, 1H, 3-Hb), 5.33 (d, *J* = 17.2 Hz, 1H, OCH₂CH=C*Ha*Hb), 5.24 (d, *J* = 10.8 Hz, OCH₂CH=CHa*Hb*), 4.65 (d, *J* = 5.6 Hz, 2H, OCH₂), 2.56-2.40 (m, 1H, 1'-H), 1.86-1.80 (m, 1H), 1.59-0.87 (m, 10H), 0.88/0.87 (2t, *J* = 7.2 Hz, 3H, CH₂CH₃) ppm. MS(EI) : 222 (M⁺, 8), 193 (9), 181 (30), 163 (69), 145 (22), 135 (98), 107 (72), 95 (76), 93 (83), 81 (67), 79 (100), 67 (82). IR (ATR): 2924s, 2857m, 1718vs, 1450m, 1277m, 1153s, 1121s, 985m, 935m cm⁻¹.

### Example 8

### 2-(4-tert-Butyl-cyclohexyl)-acrylic acid allyl ester

### Odor: green, galbanone, fruity,

Two isomers in a ratio of 1/1: ¹H-NMR (400 MHz, CDCl₃): 6.22/6.14 (2s, 1H, 3-Ha), 6.02-5.89 (m, 1H, OCH₂C*H*=CH₂), 5.56/5.50 (2s, 1H, 3-Hb), 5.35 (d, *J* = 17.1 Hz, 1H, OCH₂CH=C*Ha*Hb), 5.24 (d, *J* = 10.4 Hz, 1H, OCH₂CH=CHa*Hb*), 4.67-4.64 (m, 2H, OCH₂), 2.94-2.88/2.42-2.38 (2m, 1H, 1'-H), 1.98-1.81 (m, 3H), 1.68-1.52 (m, 2H), 1.20-0.98 (m, 4H), 0.86/0.82 (2s, 9H, C(CH3)3) ppm. MS(EI): 250 (M⁺, 2), 209 (6), 193 (56), 163 (16), 152 (22), 135 (57), 107 (76), 105 (40), 981 (46), 81 (40), 79 (100), 67 (58). IR (ATR): 2940s, 2863m, 1719vs, 1450m, 1365m, 1273m, 1237m, 1157s, 1121s, 986m, 931m cm⁻¹.

### Example 9

### 2-(3-tert-Butyl-cyclohexyl)-acrylic acid allyl ester

### Odor: earthy, woody, green, fruity

Mixture of isomers: ¹H-NMR (400 MHz, CDCl₃): 1.17/6.16 (2s, 1H, 3-Ha), 6.01-5.87 (m, 1H, OCH₂C*H*=CH₂), 5.61/5.51 (2s, 1H, 3-H), 5.37-5.29 (m, *J* = 17.2 Hz, 1H, OCH₂CH=C*Ha*Hb), 5.26-5.21 (m, 1H, OCH₂CH=CHa*Hb*), 4.62/4.58 (2d, *J* = 5.6 Hz, 2H, OCH₂), 2.49-2.40 (m, 1H, 1'-H), 1.96-0.89 (m, 9H), 0.85/0.82 (2s, 9H, *C(CH*_{*3*}*)*_{*3*}) ppm. MS(EI): 250 (M⁺, 1), 235 (1), 193 (58), 163 (18), 153 (18), 147 (20), 135 (63), 107 (77), 105 (37), 91 (53), 81 (43), 79 100), 77 (42), 67 (70). IR (ATR): 2939s, 2864m, 1720vs, 1365m, 1273s, 1151s, 1123s, 1108s, 985m, 931s cm⁻¹.

### Example 10

### 2-Cyclohex-3-enyl-acrylic acid allyl ester

### Odor: fruity, pineapple, galbanone, green

¹H-NMR (400 MHz, CDCl₃): 6.23 (s, 1H, 3-Ha), 6.03-5.93 (m, 1H, OCH₂CH=CH₂), 5.74-5.66 (m, 2H, 3',4'-H), 5.55 (s, 1H, 3-Hb), 5.35 (d, *J* = 17Hz, 1H, OCH₂CH=*Ha*Hb), 5.25 (d, *J* = 10Hz, 1H, OCH₂CH=CHa*Hb*), 4.67 (m, 2H, OCH₂), 2.81-2.73 (m, 1H, 1'-H), 2.29-2.07 (m, 3H), 1.96-1.82 (m, 2H), 1.56-1.45 (m, 1H) ppm. MS(EI): 192 (M⁺, 1), 151 (51), 133 (64), 105 (100), 91 (38), 79 (84), 67 (14), 53 (34), 41 (64). IR(ATR): 3024s, 2917m, 2839s, 1717vs, 1330m, 1266m, 1136vs, 982m, 939m cm⁻¹.

### Example 11

### 3,4,4a,5,6,7,8,8a-Octahydro-napthalene-2-carboxylic acid allyl ester

### 1,4,4a,5,6,7,8,8a-Octahydro-naphthalene-2-carboxylic acid allyl ester

### a) 3,4,4a,5,6,7,8,8a-Octahydro-napthalene-2-carbonitrile

### 1,4,4a,5,6,7,8,8a-Octahydro-naphthalene-2-carbonitrile

A mixture of octahydronaphthalen-2-one (30.4g, 0.2 mol), ZnI₂ (0.1g), KCN (39g, 0.6 mol) and trimethyl silylchloride (34.6g, 0.32 mol) in acetonitrile (40 ml) were refluxed for 25h. The brown suspension was filtered. The filtrate was concentrated *in vacuo* (the residue was destroyed with a solution of sodium hypochlorite) and redissolved in a mixture of benzene (80ml) and pyridine(200 ml). Phosphoryl chloride (153 g, 0.6 mol) was added dropwise and the resulting mixture was heated to reflux temperature for 8h. The dark solution was cooled and then poured on ice (0.5 L) and extracted with pentane. The organic phase was washed with water and brine, dried (MgSO₄) and concentrated in *vacuo.* The residue was distilled (bp 110°C/0.3 Torr) to yield 28.2 g (87%) of a colorless oil. Mixture of 4 isomers in a ratio of 1/3/10/4: ¹H-NMR (400 MHz, CDCl₃): 6.55-6.21 (4m, 1H, C*H*=CCN), 2.32-1.95 (m, 3H), 1.86-0.85 (m, 11H) ppm. GCMS (EI) isomer 1: 161 (M⁺, 79), 146 (48), 132 (42), 95 (100), 81 (27), 77 (31), 67 (52); Isomer 2: 161 (M⁺, 98), 146 (43), 132 (39), 95 (87), 82 (38), 77 (33), 67 (100); Isomer 3: 161 (M⁺, 78), 146 (45), 132 (43), 95 (100), 81 (28), 77 (36), 67 (49); Isomer 4: 161 (M⁺, 65), 146 (31), 132 (28), 95 (57), 82 (52), 77 (30), 67 (100). IR (ATR): 2923vs, 2854s, 2215m, 1634m, 1448s, 882m cm⁻¹.

### b) 3,4,4a,5,6,7,8,8a-Octahydro-napthalene-2-carboxylic acid allyl ester

### 1,4,4a,5,6,7,8,8a-Octahydro-naphthalene-2-carboxylic acid allyl ester

### Odor: fruity, sugary, little green

A solution of the carbonitrile (6.5g, 40.4 mmol, prepared in step a), acetic acid (30 ml) and conc. HCl (40 ml) was heated to reflux temperature for 7 h. The solution was then cooled to room temperature, diluted with water and extracted with ethyl acetate. The organic phase was washed with water and brine, dried (MgSO₄) and concentrated *in vacuo.* The residue was dissolved in chloroform containing some drops of dimethyl formamide. Oxalyl chloride (15.4g, 121 mmol) was added dropwise and the solution was stirred for 3 h at room temperature and was again concentrated *in* *vacuo*. The resulting acid chloride was taken up in chloroform (30 ml) and added to a solution of allyl alcohol (5.86g, 0.1 mol), pyridine (8.0g, 0.1 mol) and DMAP (50mg) in chloroform (20 ml) at 5°C. After the mixture was stirred for 3 h it was poured on water and extracted with chloroform. The organic phase was washed with aqueous HCl (1N), water and brine, dried (MgSO₄) and concentrated *in vacuo.* The residue was purified by chromatography on silica (hexane/ethyl acetate 99:1) to yield 4.9g (55%) of the allyl ester as a mixture of 4 isomers: ¹H-NMR (400 MHz, CDCl₃): 7.0-6.75 (4m, 1H, C*H*=CCO), 6.01-5.92 (m, 1H, OCH₂C*H*=CH₂), 5.36-5.21 (m, 2H, OCH₂CH=C*H*₂), 4.68-4.58 (m, 2H, OCH₂), 2.48-2.09 (m, 3H), 1.91-0.85 (m, 11H) ppm. GCMS(EI) isomer 1: 220 (M⁺, 12), 179 (17), 161 (18), 133 (100), 91 (70), 79 (15), 67 (22); Isomer 2: 220 (M⁺, 5), 179 (40), 161 (26), 135 (54), 133 (100), 91 (68), 79 (49), 67 (59); Isomer 3: 220 (M⁺, 7), 179 (44), 161 (29), 133 (100), 91 (65), 79 (47), 67 (53); Isomer 4: 220 (M⁺, 17), 179 (50), 163 (44), 161 (40), 133 (100), 91 (62), 81 (46), 79 (51), 67 (68). IR (ATR): 2923sm, 2853m, 1711vs, 1647m, 1447m, 1234vs, 1061s, 989m, 928m cm⁻¹.

### Example 12

### Composition for Men's toiletries

| | |
|---|---|
| Linalyl acetate | 65 |
| Decyl aldehyde FCC (1% DEP) | 10 |
| Allyl amyl glycolate | 15 |
| Ambrofix | 10 |
| Lemon ess. italie orpur | 90 |
| Cyclohexal | 17 |
| Damascone alpha (10% in PE) | 13 |
| Dihydromyrcenol | 150 |
| Dipropylene glycol | 224 |
| Evernyl | 4 |
| Fixolide | 70 |
| Geranium ess. afrique | 9 |
| Hedione | 55 |
| Iso E Super | 32 |
| Lavandin grosso ess. orpur | 45 |
| Linalol synth. | 30 |
| Menthol naturel (10% in DEP) | 15 |
| Methylionantheme 100% | 18 |
| Oranger Crist | 3 |
| Precyclemone B | 15 |
| Sandalore | 5 |
| Tricyclal (10% in PE) | 15 |
| Vertofix coeur | 80 |
| 2-Cyclohexyl-acrylic acid allyl ester | 10 |
| | 1000 |

In this men's fragrance accord, 2-cyclohexyl-acrylic acid allyl ester reinforces the fresh and clean aspect. It provides richness and volume and increases the diffusivity of this Fougère fragrance. It enhances the sparkling effect of the green hesperidic and agrestic top notes and harmonizes with the floral, musky, ambery and woody middle and dry-down notes.

### Example 13

### Fresh lavandine composition for APC (all purpose cleaner)

| | |
|---|---|
| Fenchyl acetate | 50 |
| Nopyle acetate | 70 |
| Verdyle acetate | 55 |
| Fenchole (10% in BB) | 5 |
| Amyl vinyl carbinol | 2 |
| Borneol cryst. | 6 |
| Camphor synth. | 35 |
| Coumarine | 45 |
| Dihydro myrcenol | 380 |
| Elemi resinoid (50% in BB) | 70 |
| Ethyl isoamyl ketone | 10 |
| Eucalyptol | 60 |
| Methyl heptenone | 53 |
| Myrcene | 2 |
| Orange terpenes | 50 |
| p-Cymene | 1 |
| alpha-Pinene | 6 |
| Verdyl propionate | 35 |
| gamma-Terpinene | 3 |
| Terpinolene | 2 |
| Tetrahydro linalool | 50 |
| Thymol cryst. | 4 |
| Veloutone | 5 |
| 2-Cyclohexyl-acrylic acid allyl ester | 1 |
| | 1000 |

The green-galbanum note of 2-cyclohexyl-acrylic acid allyl ester gives a fresh and clean effect in the fragrance and enhances the lavandine aspect in this composition. 2-Cyclohexyl-acrylic acid allyl ester blends nicely with agrestic, hesperidic and fruity notes.

## Claims

1. Compounds of the formula I 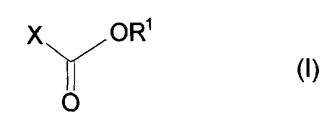 wherein R¹ is a straight or branched C₃-C₄ alkenyl residue having a double bond in position 2 or a C₄ alkylcycloalkyl residue,
X is either a residue of formula A, 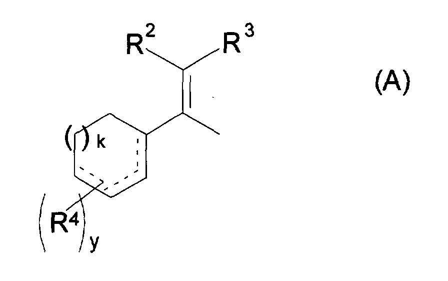
wherein
R² and R³ are independently hydrogen, methyl or ethyl,
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k is 0 or an integer of 1,2 or 3,
and the dotted lines in formula A stand for one optional double bond;
or X is a residue of formula B 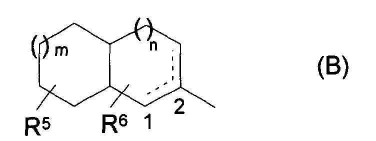 wherein
R⁵ or R⁶ are independently hydrogen or a straight or branched C₁-C₃ hydrocarbon residue being at any position of the ring system B, and the sum of the carbon atoms of R⁵ and R⁶ is 3 or less,
n is 0 or an integer of 1 or 2,
m is 0 or an integer of 1 or 2,
and the dotted lines in formula B stand for an optional double bond either in position 1 or 2.

2. Compounds according to claim 1
wherein R¹ is a C₃ allyl
and
X is a residue of formula A 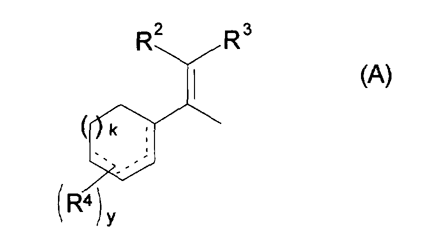
wherein
R² and R³ are independently hydrogen or methyl,
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k is 0 or 1.

3. Compounds according to claim 1
wherein R¹ is C₄ alkylcycloalkyl or a C₄ alkenyl residue having the double bond in position 2,
and
X is a residue of formula (A) 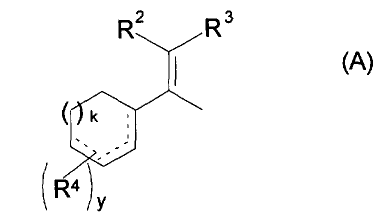
wherein
R² and R³ are independently hydrogen or methyl,
one or more hydrogen atoms in the ring system A may be substituted by (R⁴)_{y}, R⁴ is a straight or branched C₁-C₄ hydrocarbon residue,
y is an integer of 1 to 4,
and the sum of the carbon atoms of all residues R⁴ is 4 or less,
k is 0 or 1.

4. A composition with fruity organoleptic characteristics comprising a compound according to any of the preceding claims.

5. Composition according to claim 4 comprising one or more additional organoleptic ingredients.

6. Fragrance composition comprising a compound according to any of the preceding claims.

7. Flavor composition comprising a compound according to any of the preceding claims.

8. Composition according to any of the preceding claims exhibiting a green galbanum fresh and fruity-pineapple odor.

9. Consumer product comprising a composition according to any of the preceding claims.
